# EUROPEAN PATENT SPECIFICATION

(11) **EP 3 714 886 B1**
(45) Date of publication and mention of the grant of the patent: **18.03.2026**
(21) Application number: 18892237.1
(22) Date of filing: 20.12.2018
(51) Int. Cl.: A61K 31/506, A61P 39/00, A61P 11/00

(54) **GSK3 INHIBITORS FOR PULMONARY FIBROSIS INDUCED BY RADIATION OR DRUG THERAPY FOR ANTICANCER TREATMENT**
GSK3-INHIBITOREN FÜR PULMONALE FIBROSE, DIE DURCH STRAHLUNGS- ODER ARZNEIMITTELTHERAPIE ZUR ANTIKREBSBEHANDLUNG INDUZIERT WIRD
INHIBITEURS DE GSK3 POUR LA FIBROSE PULMONAIRE INDUITE PAR RADIATION OU THÉRAPIE MÉDICAMENTEUSE POUR LE TRAITEMENT ANTICANCÉREUX

(30) Priority: 20.12.2017 KR 20170175870; 20.12.2017 KR 20170175947
(43) Date of publication of application: 30.09.2020
(73) Proprietor: Korea Institute of Radiological & Medical Sciences, Seoul 01812 (KR)
(72) Inventor: LEE, Yoon-Jin, Seoul 03968 (KR); SEO, Heang Ran, Seongnam-si, Gyeonggi-do 13488 (KR); LEE, Hae-Jun, Seoul 01784 (KR); KIM, A-Ram, Uijeongbu-si, Gyeonggi-do 11636 (KR); CHOI, Inhee, Seongnam-si, Gyeonggi-do 13488 (KR); KANG, Sunhee, Seongnam-si, Gyeonggi-do 13488 (KR); NAM, Jae-kyung, Seoul 01752 (KR)
(74) Representative: Gassner, Birgitta
(86) International application number: PCT/KR2018/016369
(87) International publication number: WO 2019/125016

(56) References cited:
- WO-A1-2006/100490
- WO-A2-01/47533
- WO-A2-2006/034207
- THOTALA D K ET AL: "A New Class of Molecular Targeted Radioprotectors: GSK-3beta Inhibitors", INTERNATIONAL JOURNAL OF RADIATION: ONCOLOGY BIOLOGY PHYSICS, PERGAMON PRESS, USA, vol. 76, no. 2, February 2010 (2010-02-01), pages 557 - 565, XP026852871, ISSN: 0360-3016, [retrieved on 20100113]
- JIANG XIAOYU ET AL: "A GSK-3[beta] Inhibitor Protects Against Radiation Necrosis in Mouse B", INTERNATIONAL JOURNAL OF RADIATION: ONCOLOGY BIOLOGY PHYSICS, PERGAMON PRESS, USA, vol. 89, no. 4, 20 June 2014 (2014-06-20), pages 714 - 721, XP028870533, ISSN: 0360-3016, DOI: 10.1016/J.IJROBP.2014.04.018
- REN X ET AL: "GSK3[beta] Inhibitor, SB415286, Is a Radioprotector for Normal Lung", INTERNATIONAL JOURNAL OF RADIATION: ONCOLOGY BIOLOGY PHYSICS, vol. 96, no. 2, 1 October 2016 (2016-10-01), XP029747355, ISSN: 0360-3016, DOI: 10.1016/J.IJROBP.2016.06.2085
- GURRIERI CARMELA ET AL: "3-(2,4-Dichlorophenyl)-4-(1-methyl-1H-indol-3-yl)-1H-pyrrole-2,5-dione (SB216763), a Glycogen Synthase Kinase-3 Inhibitor, Displays Therapeutic Properties in a Mouse Model of Pulmonary Inflammation and Fibrosis", JOURNAL OF PHARMACOLOGY AND EXPERIMENTAL THERAPEUTICS, vol. 332, no. 3, March 2010 (2010-03-01), pages 785 - 794, XP009529441, ISSN: 0022-3565
- SONG YEONHWA ET AL: "Identification of radiation-induced EndMT inhibitors through cell-based phenomic screening.", FEBS OPEN BIO 01 2019, vol. 9, no. 1, January 2019 (2019-01-01), pages 82 - 91, XP009529428, ISSN: 2211-5463
- LI LI ET AL: "Anticancer therapy and lung injury: molecular mechanisms", EXPERT REVIEW OF ANTICANCER THERAPY, vol. 18, no. 10, 3 October 2018 (2018-10-03), GB, pages 1041 - 1057, XP093273636, ISSN: 1473-7140, DOI: 10.1080/14737140.2018.1500180
- WANG, X.: "Pharmacologically blocking p53-dependent apoptosis protects intestinal stem cells and mice from radiation", SCIENTIFIC REPORTS, vol. 5, 2015, XP055621804
- BAARSMA, H. A.: "Glycogen synthase kinase-3(GSK-3) regulates TGF-b1- induced differentiation of pulmonary fibroblasts", BRITISH JOURNAL OF PHARMACOLOGY, vol. 169, no. 3, 2013, pages 590 - 603, XP055621806

## Description

### Technical Field

The present invention relates to a pharmaceutical composition comprising specific GSK3 inhibitors for use in preventing or treating pulmonary fibrosis, wherein the pulmonary fibrosis is induced by radiation exposure or drug therapy for anticancer treatment.

### Background Art

There are various cases of biological damage caused by radiation exposure such as radiation exposure accidents at industrial sites dealing with radiation, side effects on normal tissues caused by radiation treatment, *etc.*

Radiation exerts an adverse effect on cells and tissues primarily due to cytotoxicity. Human exposure to ionizing radiation occurs mostly through anti-cancer radiotherapy, or occupational or environmental exposure.

Radiation exposure may occur in occupational settings, e.g., exposure (or potential exposure) to radiation in the industry of nuclear power or nuclear weapons. Currently, there are 104 commercially-licensed nuclear power plants in the U.S.A. Globally, a total of 430 nuclear power plants are in operation in 32 countries. All employees hired in these nuclear power plants may be exposed to radiation when carrying out their assigned duties. The Three Mile Island Nuclear Power Plant Accident occurred on March 28, 1979, when radioactive materials were released into the nuclear reactor building and the surrounding environment, shows the potential for very hazardous exposure. Even without such a major accident, workers serving in the nuclear power industry are being exposed to higher levels of radiation compared to civilians.

Other causes of occupational exposure may arise from smoke alarm, emergency signs, other consumables, and solvents remaining in the manufacture of mechanical components, plastics, and radioactive medical products.

Further, biological damage due to radiation exposure is often developed as chronic side effects upon radiation treatment, and this lowers the recovery rate of radiation treatment. Recently, according to the development of the technique for radiation treatment, the survival rate of cancer patients who received a radiotherapy is getting higher, but biological damages occurred as side effects due to radiation, especially pulmonary fibrosis, pose a major problem that deteriorates the quality of life of cancer patients. With the recent development of radiotherapy equipment and software and the evolution of the radiation biological concept, radiotherapy techniques have been developed, that are capable of effectively controlling only cancer lesions while protecting normal tissues with one to several times (five times or so) of radiation treatments. However, these techniques are still restrictively used depending on the phase of cancer progression, the site of cancer formation, *etc.*

Despite the development of such radiotherapy techniques, side effects such as pulmonary fibrosis which inevitably occurs upon radiation treatment are frequently shown in patients treated with chest radiation. Radiation pneumonia occurs after 2 to 3 months of radiotherapy in 10% to 15% of patients who received the chest radiotherapy for the treatment of lung cancer, breast cancer, or Hodgkin lymphoma, and is developed to a fibrotic disease, which is a chronic side effect, after 6 months. Pulmonary fibrosis progressed in such a way would still be remained even after about 2 years, and as result, the decline of lung function, patient's pain, and discomfort in life are accompanied. (Xie H et al., Exp Biol Med (Maywood):238(9):1062-8. 2013.).

The GSK-3 inhibitor CHIR99021 has been identified as an intestinal radioprotector in mice and humans by selectively blocking p53-dependent apoptosis and PUMA induction in intestinal stem and progenitor cells (Wang X et al., Sci Rep. 2015 Apr 10:5 8566). WO2006034207A describes methods involving the use of GSK3 inhibitors in combination with radiation therapy. These methods may result in an amelioration of one or more of cognitive decline, toxicity to vascular endothelial cells, and neuron death associated with radiation therapy. Ren X *et al.* disclose that a GSK3β inhibitor SB415286 is a radioprotector for normal lung (Ren X et al., International Journal of Radiation Oncology Biology Physics, 96(2)). Gurrieri C. *et al.* describe that the GSK-3 inhibitor SB216763 prevents lung inflammation and the subsequent fibrosis caused by bleomycin, an anticancer agent (Gurrieri C. et al., J Pharmacol Exp Ther. 2010 Mar;332(3):785-94). Baarsma H.A. *et al.* disclose that glycogen synthase kinase-3 (GSK-3) regulates TGF-β₁-induced differentiation of pulmonary fibroblasts (Baarsam H.A. et al., r J Pharmacol. 2013 Jun;169(3):590-603).

Currently, immunosuppressants are primarily used for the treatment of pulmonary fibrosis. Steroids or cytotoxic drugs, *etc.,* may be used, but steroids are used first. In addition, as therapeutic agents for pulmonary fibrosis caused by radiation exposure, a combination therapy of steroids, azathioprine, or cyclophosphamide is currently used (Ochoa et al., Journal of Medical Case Reports, 6:413.2012). However, there is no evident ground that such treatments improve the patients' survival rates or their quality of life. In addition, up to date, several fibrosis inhibitors have been attempted in animal experiments and in small-scale patients, but no noticeable effect has been demonstrated.

Accordingly, there is an urgent demand for the development of radioprotectants or medicines for mitigating radiation, which are capable of protecting or mitigating tissue damages caused by radiation (e.g., pulmonary fibrosis caused by radiation).

### Disclosure

### Technical Problem

The present inventors have made intensive efforts to develop such radioprotectants or medicines for mitigating radiation, and as a result, they have newly identified that compounds *(i.e.,* N-6-[2-[[4-(2,4-dichlorophenyl)-5-(1H-imidazol-2-yl)-2-pyrimidinyl]amino]ethyl]-3-nitro- 2,6-pyridinediamine and 6-[[2-[[4-(2,4-dichlorophenyl)-5-(5-methyl-1H-imidazol-2-yl)- 2-pyrimidinyl]amino]ethyl]amino]-3-pyridinecarbonitrile), which are known as GSK inhibitors, have excellent radioprotection or radiomitigation effects, thereby completing the present invention.

### Technical Solution

An object of the present invention is to provide a pharmaceutical composition for use in preventing or treating pulmonary fibrosis induced by radiation exposure or drug therapy for anticancer treatment, comprising the above mentioned GSK3 inhibitors.

The aspects of the invention are as recited in the appended set of claims. Irrespective of any statement made in the remainder of the description, the embodiments of the invention are solely as detailed in the appended set of claims.

### Advantageous Effects

The GSK3 inhibitor of the present invention has an excellent effect of preventing or mitigating radiation-induced damage which is a side effect including vascular damage, skin damage, gastrointestinal tract damage, tissue inflammation, and tissue fibrosis, which are shown upon radiation exposure.

Further, the GSK3 inhibitor of the present invention has an excellent effect of preventing or treating pulmonary fibrosis, particularly, pulmonary fibrosis caused by radiation exposure or drug therapy for anticancer treatment. Accordingly, it is expected that the pharmaceutical composition of the present invention can be effectively used for preventing or treating pulmonary fibrosis induced by radiation exposure or drug therapy for anticancer treatment.

In addition, the GSK3 inhibitor of the present invention has an effect of effectively preventing or treating biological damage, which can be exhibited upon radiation treatment, by inhibiting DNA damage.

### Brief Description of Drawings

### <Description of Symbols of Drawings>

No IR: control group (non-irradiation group)
IR: irradiation group
IR + CHIR-99021 (or CHIR-98014): irradiation and CHIR-99021 (or CHIR-98014) treatment group
No Bleomycin: control group (Bleomycin non-injection group)
Bleomycin: Bleomycin injection group
Bleomycin + CHIR-99021 (or CHIR-98014): Bleomycin injection and CHIR-99021 (or CHIR-98014) treatment group

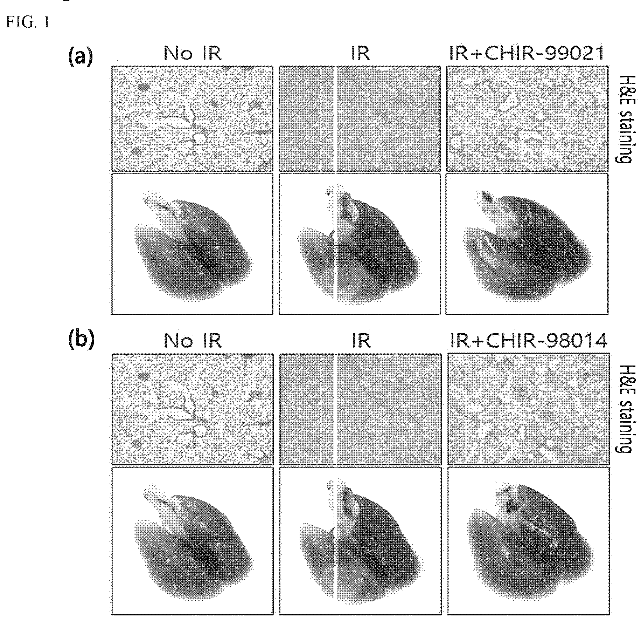

FIG. 1 shows photographs of the results confirming the inflammatory response and fibrosis of the tissue damage sites of a mouse animal model by a hematoxylin and eosin staining method after treatment or non-treatment with the GSK3 inhibitor (FIG. 1a: CHIR-99021 and FIG. 1b: CHIR-98014) of the present invention at a concentration of 30 mg/kg one hour prior to irradiation, which was followed by irradiation of the site of the lung of the mouse animal model with an intensity of 90 Gy.

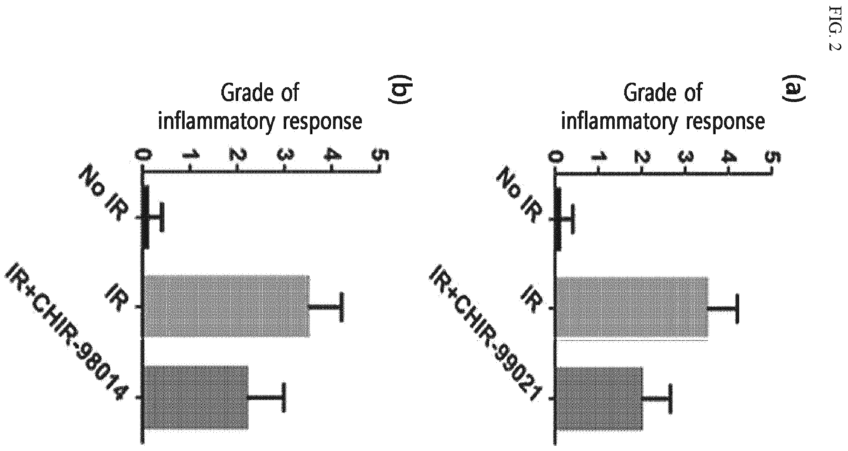

FIG. 2 illustrates graphs which statistically show the degree of the inflammatory response in FIG. 1 by grading.

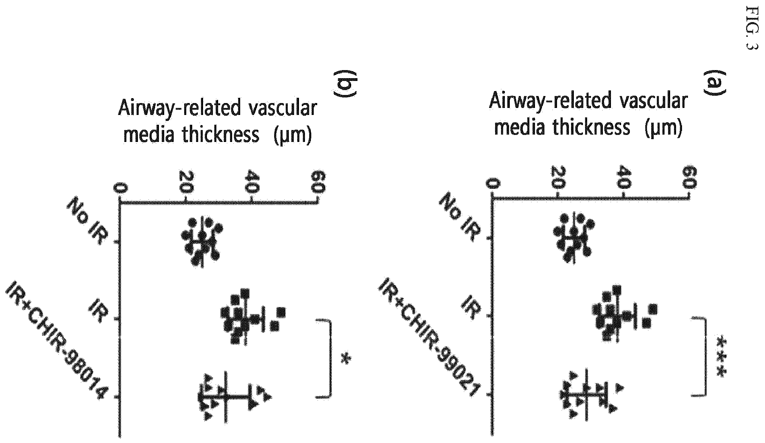

FIG. 3 illustrates graphs which show changes in the thickness of the vascular wall of a mouse animal model compared to the non-treatment group (IR) when the mouse animal model was treated with CHIR-99021 (FIG. 3a) or CHIR-98014 (FIG. 3b) together with irradiation of the chest.

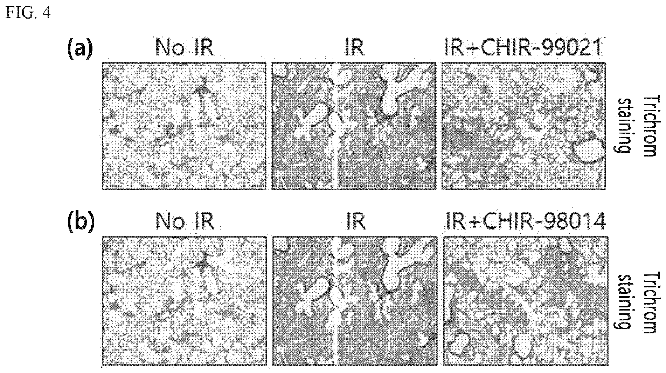

FIG. 4 shows photographs of the results confirming collagen in the vascular endothelial sites of the lung of a mouse animal model by a trichrome staining method after treatment or non-treatment with CHIR-99021 (FIG. 4a) or CHIR-98014 (FIG. 4b) in the mouse animal model, which was followed by irradiation of the site of the lung.

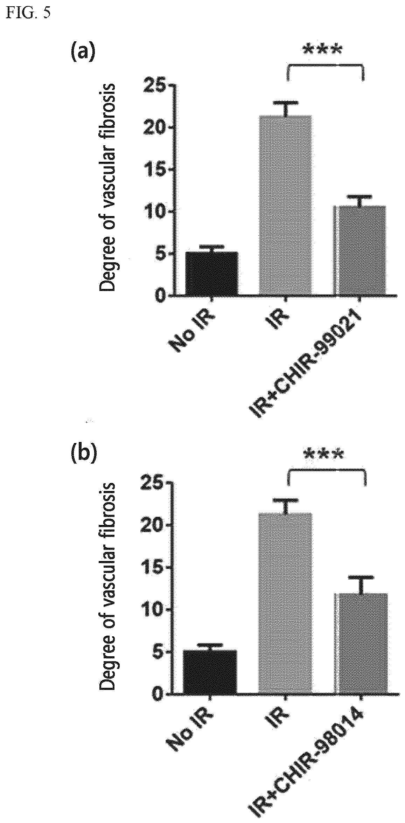

FIG. 5 illustrates graphs which statistically show the level of expression of collagen *(i.e.,* a molecule associated with pulmonary fibrosis) of FIG. 4 by carrying out a trichrome staining method.

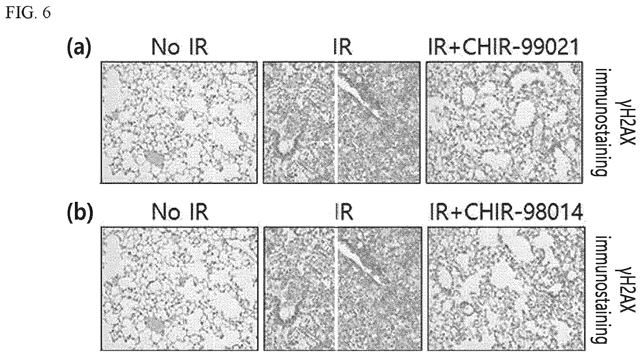

FIG. 6 shows photographs of the results confirming DNA damage by a γH2AX immunostaining method after treatment or non-treatment with CHIR-99021 (FIG. 6a) or CHIR-98014 (FIG. 6b) at a concentration of 30 mg/kg in a mouse animal model one hour prior to irradiation, which was followed by irradiation of the site of the lung of the mouse animal model with an intensity of 90 Gy.

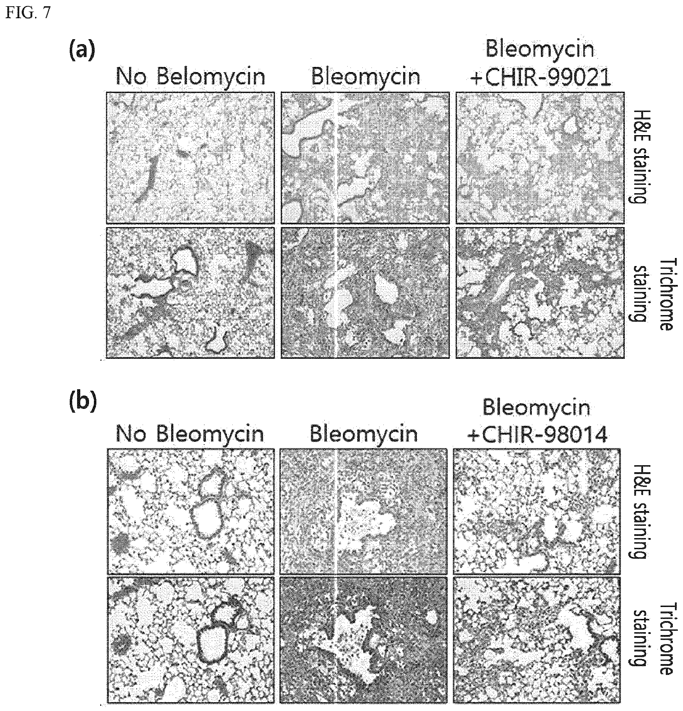

FIG. 7 shows photographs of the results confirming the lung tissue of a mouse animal model by hematoxylin and eosin staining and trichrome staining methods after treatment or non-treatment with CHIR-99021 (FIG. 7a) or CHIR-98014 (FIG. 7b) at a concentration of 30 mg/kg in the mouse animal model prior to bleomycin injection.

### Best Mode for Carrying Out the Invention

The references to the methods of treatment of this description are to be interpreted as references to compounds, pharmaceutical compositions and medicaments of the present invention for use in a method for treatment of the human (or animal) body by therapy. All of the terms used herein, unless otherwise defined, are used as meaning the same as commonly understood by one of ordinary skill in the art to which this invention belongs.

Described herein is a pharmaceutical composition for inhibiting DNA damage, comprising a GSK3 inhibitor; a pharmaceutical composition for protecting or mitigating radiation against radiation-induced damage, comprising a GSK3 inhibitor; and a pharmaceutical composition for preventing or treating pulmonary fibrosis, comprising a GSK3 inhibitor.

The GSK3 inhibitor be included without limitation as long as it shows an effect of inhibiting DNA damage or an effect of preventing or treating radiation-induced damage or pulmonary fibrosis. Specifically, the GSK3 inhibitor of the present invention may be N-6-[2-[[4-(2,4-dichlorophenyl)-5-(1H-imidazol-2-yl)-2-pyrimidinyl] amino]ethyl]-3-nitro-2,6-pyridinediamine, 6-[[2-[[4-(2,4-dichlorophenyl)-5-(5-methyl- 1H-imidazol-2-yl)-2-pyrimidinyl]amino]ethyl]amino]-3-pyridinecarbonitrile), or a pharmaceutically acceptable salt or solvate thereof, but is not limited thereto.

The "DNA damage" above is induced by radiation exposure or drug therapy for anticancer treatment, and may refer to damage that may be caused by the direct loss of a structure or function of DNA or to damage which may indirectly act on DNA. The DNA damage above may accompany any symptoms of biological damage; specifically, the DNA damage above may accompany symptoms of vascular damage, skin damage, gastrointestinal tract damage, tissue inflammation, or tissue fibrosis, *etc.,* or may accompany symptoms of pulmonary fibrosis, *etc.,* but the DNA damage is not limited thereto.

The GSK3 inhibitor above may be a compound of Formula 1 and/or Formula 2 below, or a pharmaceutically acceptable salt or solvate thereof.

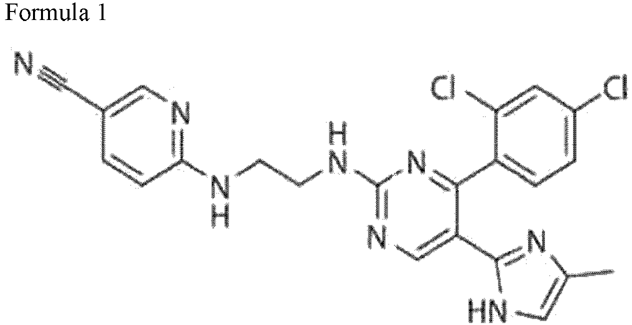

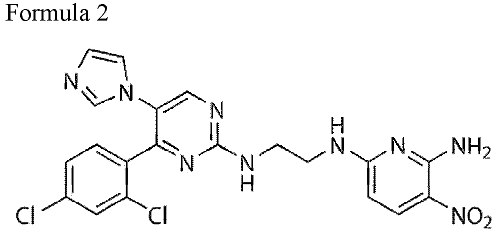

The chemical name of the compound of Formula 1 above is 6-[[2-[[4-(2,4-dichlorophenyl)-5-(5-methyl-1H-imidazol-2-yl)-2-pyrimidinyl]amino]ethyl]amino]-3-pyridinecarbonitrile), and is also disclosed as "CHIR-99021" or "CT99021".

The chemical name of the compound of Formula 2 above is (N-6-[2-[[4-(2,4-dichlorophenyl)-5-(1H-imidazol-2-yl)-2-pyrimidinyl]amino]ethyl]-3-nitro-2,6-pyridinediamine), and is also disclosed as "CHIR-98014".

The compounds of Formula 1 and Formula 2 above, as GSK3 inhibitors, are known as components that can be added into a stem cell culture medium (Korean Laid-open Patent Publication No. 10-2014-0076935), but their effects relevant to DNA damage inhibition, radiation damage protection, or pulmonary fibrosis are not known at all.

In one specific embodiment, as a result of measuring the level of DNA damage in normal cells around the irradiated site after irradiating a mouse, it was confirmed that DNA damage was remarkably reduced in the mouse administered with the compound of Formula 1 or the compound of Formula 2, as compared to the control group.

As used herein, the term "radioprotection" refers to the inhibition or alleviation of any radiation-induced damage, which is induced by radiation exposure, by applying to the body prior to the radiation exposure.

As used herein, the term "radiomitigation" refers to the inhibition or alleviation of any radiation-induced damage, which is induced by radiation exposure, by applying to the body within a short period of time after radiation exposure before appearing distinct signs of the radiation exposure.

As used herein, the term "within a short period of time" refers to a period of time during which any radiation-induced damage can be inhibited or alleviated by applying before appearing distinct signs of damage caused by radiation exposure. In one specific embodiment, the term "within a short period of time" refers to a period of time within 36 hours, within 24 hours, or within 12 hours after irradiation, but is not limited thereto.

As used herein, the term "radiation-induced damage" refers to any biological damage caused by radiation exposure. For example, the radiation-induced damage includes vascular damage, skin damage, gastrointestinal tract damage, tissue inflammation, or tissue fibrosis, but is not limited thereto.

As used herein, the term "pharmaceutically acceptable salt" includes salts that are commonly used in the field of pharmaceuticals, such as hydrochloride, hydrobromide, hydroiodide, hydrofluoride, sulfate, sulfonate, citrate, camphorate, maleate, acetate, lactate, nikitinate, nitrate, succinate, phosphate, malonate, malate, salicylate, phenylacetate, stearate, formate, fumarate, urea, sodium, potassium, calcium, magnesium, zinc, lithium, cinnamate, methylamino, mathanesulfonate, picrate, p-toluenesulfonate, naphthalenesulfonate, tartrate, triethylamino, dimethylamino, and tri(hydroxylmethyl)aminomethane, and specifically, the pharmaceutically acceptable salt may be hydrochloride, but the pharmaceutically acceptable salt is not limited thereto.

As used herein, the term "solvate" or "pharmaceutically acceptable solvate" above refers to a solvate formed from the conjugation of one or more solvent molecules with the compound of Formula 1 or Formula 2. The term "solvate" includes a hydrate (e.g., hemihydrate, monohydrate, dehydrate, trihydrate, tetrahydrate, *etc.).*

As used herein, the term "pulmonary fibrosis" may be pulmonary fibrosis occurred due to various reasons, specifically, due to radiation exposure, drug therapy for anticancer treatment, smoking, or dusty work environments, *etc.,* but is not limited thereto. Further, the pulmonary fibrosis above may be side effects of radiotherapy occurred by exposure of radiation to normal tissues during radiotherapy for cancer or may be side effects of drug therapy for anticancer treatment, but is not limited thereto. The radiotherapy or drug therapy for cancer, which is capable of inducing pulmonary fibrosis, includes treatment of a lung cancer, a breast cancer, or Hodgkin lymphoma, *etc.,* but is not limited thereto.

The GSK3 inhibitor of the present invention may exhibit an effect of protecting or mitigating radiation or an effect of preventing or treating pulmonary fibrosis by inhibiting DNA damage caused by radiation.

As used herein, the term "prevention" refers to any activity that inhibits, alleviates, or delays the symptoms due to DNA damage *(i.e.,* radiation damage and/or lung fibrosis) by administering the compositions of the present invention to a subject.

As used herein, the term "treatment" refers to any activity that improves or benefits the symptoms due to DNA damage (*i.e.,* radiation damage and/or pulmonary fibrosis symptoms) by administering the compositions of the present invention to a subject.

Another aspect of the present invention is to provide a method for inhibiting DNA damage, comprising administering the pharmaceutical compositions.

Still another aspect of the present invention is to provide a method for protecting or mitigating radiation against radiation-induced damage, comprising administering the pharmaceutical compositions.

Still another aspect of the present invention is to provide a method for preventing or treating pulmonary fibrosis, comprising administering the pharmaceutical compositions.

The radiation-induced damage, pulmonary fibrosis, prevention, and treatment are as described above.

As used herein, the term "administration" refers to the introduction of the compositions of the present invention to a subject by any suitable method, and the administration route can be administered through various routes of oral or parenteral administration as long as it can reach a target tissue.

The pharmaceutical compositions above may be administered before or after exposure to a DNA damage-inducing factor. The DNA damage-inducing factor may be radiation, drug administration for anticancer treatment, *etc.,* but is not limited thereto. Specifically, the pharmaceutical compositions above may be administered before, after, or simultaneously with radiation exposure, and may be administered before, after, or simultaneously with drug therapy for anticancer treatment, and specifically, may be administered before radiation exposure, but it is not limited thereto.

A subject to which the pharmaceutical compositions are administered may be all animals including humans. The animals above may be mammals such as humans as well as cattle, horses, sheep, pigs, goats, camels, antelopes, dogs, cats, *etc.,* which are in need of treatment of similar symptoms. Further, the animals may refer to those other than humans, but are not limited thereto.

The pharmaceutical compositions according to the present invention, for the purpose of achieving the effect of protecting or mitigating radiation, can be administered by dividing the administration into several times such that the total dosage per day reaches about 0.1 mg/kg to 100 mg/kg as the compound of Formula 1, based on adults. The dosage above may be appropriately increased or decreased depending on the intensity of radiation causing damage, type of radiation-induced damage, or degree of progression, administration route, gender, age, weight, *etc.*

In one specific embodiment of the present invention, it was confirmed that tissue damage, vascular damage, and pulmonary fibrosis, which are caused by irradiation, were suppressed in a mouse model administered with the compound of Formula 1 or the compound of Formula 2. Accordingly, the compositions of the present invention, which comprise a GSK3 inhibitor, may be used for protecting or mitigating radiation, or for preventing or treating pulmonary fibrosis.

Still another aspect of the present invention is to provide use of a GSK3 inhibitor for inhibiting DNA damage.

Still another aspect of the present invention is to provide use of a GSK3 inhibitor for protecting or mitigating radiation against radiation-induced damage.

Still another aspect of the present invention is to provide use of a GSK3 inhibitor for preventing or treating pulmonary fibrosis.

The GSK3 inhibitor, radiation-induced damage, pulmonary fibrosis, prevention, and treatment are as described above.

### Detailed Description of the Invention

Hereinafter, the present invention will be described in detail through exemplary embodiments below. However, these exemplary embodiments are for illustrative purposes only and are not intended to limit the scope of the present invention.

### Examples

### Experimental Method

### (1) Hematoxylin and Eosin staining (H&E staining) method for biological tissues

The tissue of a mouse was fixed with 10% neutral formalin for one day, and a paraffin section was made. In order to remove the paraffin around the tissue, the tissue was allowed to react with a xylene solution and 95%, 90%, and 70% ethanol solutions in sequential order for 5 minutes each, and then immersed in a hematoxylin solution for 1 minute to stain the nuclei and then washed under running water for 10 minutes. Thereafter, the cytoplasm was stained by immersing the tissue in an eosin solution for 30 seconds. Thereafter, the tissue was immersed in 50%, 70%, 90%, and 95% ethanol solutions, and a xylene solution in sequential order, followed by dropping one drop of a mounting solution. Then, the tissue was covered with a cover slide and observed under a microscope (Carl Zeiss Vision).

### (2) Trichrome staining method for biological tissues

The tissue of a mouse was fixed with 10% neutral formalin for one day, and a paraffin section was made. In order to remove paraffin around the tissue, the tissue was allowed to react with a xylene solution and 95%, 90%, and 70% ethanol solutions in sequential order for 5 minutes each. For the antigen activation of the tissue, the tissue was immersed in a 0.1 M citric acid solution (pH 6.0) and then boiled for 20 minutes. Subsequently, the tissue was allowed to react with a Bouin's solution for 1 minute, Weigert's hematoxylin for 10 minutes, phoshotunstic/phosphomolydic acid for 10 minutes, aniline blue for 5 minutes, and 1% acetic acid for 1 minute in sequential order. Thereafter, a dehydration process was performed, and then the tissue was covered with a cover glass and observed under a microscope (Carl Zeiss Vision).

### (3) γH2AX immunostaining method

The tissue of a mouse was fixed with 10% neutral formalin for one day, and a paraffin section was made. In order to remove paraffin around the tissue, the tissue was immersed in a xylene solution and 100%, 95%, 90%, and 70% ethanol solutions in sequential order. For the antigen activation of the tissue, the tissue was immersed in a 0.1 M citric acid solution (pH 6.0) and boiled for 30 minutes, and then reacted with 3% hydrogen peroxide for 15 minutes. The tissue was allowed to react with γH2AX (abcam), which was diluted in 1:200 ratio in a PBS solution (including phosphate based saline buffer and 0.1% triton x-100), at 4°C for 16 hours. After washing the tissue with PBS, a secondary antibody conjugated with biotin was diluted in 1:200 ratio and reacted with the tissue at room temperature for 30 minutes. After reacting the tissue with ABC (Avidin biotin complex) at room temperature for 30 minutes and color-developing 3,3'-DAB (3,3'-diaminobenzidine), the tissue was counterstained with hematoxylin. Subsequently, the tissue was immersed in 50%, 70%, 90%, 95%, and 100% ethanol solutions and a xylene solution in sequential order, followed by dropping one drop of a mounting solution. Then, the tissue was covered with a cover slide and observed under a microscope (Carl Zeiss Vision).

### Example 1

### Inhibition test of GSK3 inhibitor on tissue damage caused by radiation

The lung tissue of a mouse animal model, in which the chest of the mouse was irradiated in a size of 3 mm with an intensity of 90 Gy, was fixed with 10% formalin, and a paraffin section was made. Thereafter, tissue inflammatory response and fibrosis were identified by using a hematoxylin and eosin staining method. Upon observing the tissue with this staining method, the cell nucleus is observed in blue and the cytoplasm in pink. One hour before irradiating the mouse animal model, 30 mg/kg of CHIR-99021 or CHIR-98014 was administered intraperitoneally. Two weeks after the irradiation, the inflammatory response and fibrosis of the tissue damage site were identified by using a hematoxylin and eosin staining method.

FIG. 1 shows photographs of the results confirming the inflammatory response and fibrosis of the tissue damage sites of the mouse animal model by a hematoxylin and eosin staining method after treatment or non-treatment with the GSK3 inhibitor at a concentration of 30 mg/kg one hour prior to irradiation, which was followed by irradiation of the site of the lung of the mouse animal model with an intensity of 90 Gy.

As shown in FIG. 1, when compared to the normal tissue (No IR) through the hematoxylin and eosin staining, it was confirmed that the inflammatory cells infiltrated the tissue of the 90 Gy irradiation group (IR). Further, in the tissue of the 90 Gy irradiation group (IR), it can be identified that fibrotic matrix appeared around the damaged blood vessels as compared to the normal tissue. In contrast, in the tissue of the mouse (IR+CHIR-98014; FIG. 1b) treated with CHIR-99021 (IR+CHIR-99021; FIG. 1a) or CHIR-98014, it was confirmed that the infiltration of the inflammatory cells and the fibrotic matrix around blood vessels were remarkably reduced as compared to the non-treatment group (IR).

FIG. 2 illustrates graphs which statistically show the degree of the inflammatory response shown in FIG. 1 by grading.

Based on the results above, it can be identified that the treatment with CHIR-99021 or CHIR-98014 remarkably reduced the tissue damage caused by radiation.

### Example 2

### Inhibition test of GSK3 inhibitor on in vivo vascular damage caused by radiation

The lung tissue of a mouse animal model, in which the chest of the mouse was irradiated in a size of 3 mm with an intensity of 90 Gy, was fixed with 10% formalin, and a paraffin section was made. Thereafter, the tissue was stained with hematoxylin and eosin. Upon observing the tissue with this staining method, the cell nucleus is observed in blue and the cytoplasm in pink. One hour before irradiating the mouse animal model, 30 mg/kg of CHIR-99021 or CHIR-98014 was administered intraperitoneally. Two weeks after the irradiation, the change in the thickness of the vascular wall according to the vascular damage in the lung tissue of the mouse animal model was confirmed by a hematoxylin and eosin staining method. The results are statistically shown in FIG. 3.

As shown in FIG. 3, when compared to the normal tissue (No IR), the thickness of the vascular wall of the irradiation group (IR) was considerably increased due to the vascular damage according to the irradiation; further, upon treatment with CHIR-99021 or CHIR-98014, it was observed that the thickness of the vascular wall of the irradiation group (IR) was remarkably reduced compared to the non-treatment group (IR).

Based on the results above, it was confirmed that the vascular damage progressed after irradiating the mouse and that the vascular damage caused by irradiation was remarkably inhibited by the treatment with CHIR-99021 or CHIR-98014.

### Example 3

### Pulmonary fibrosis inhibition test in experimental animal model

In order to observe whether a phenomenon of pulmonary fibrosis of vascular endothelial cells is shown as the symptom of pulmonary fibrosis occurred after radiotherapy, the site of the lung of a C57BL/6 mouse was topically irradiated with an intensity of 90 Gy. After excising the lung from the mouse, the cross-section of the aorta of the lung tissue was stained by a trichrome staining method in order to identify collagen which is a protein shown upon fibrosis of the pulmonary vascular endothelial cells. The CHIR-99021 or CHIR-98014 treatment group was administered intraperitoneally in an amount of 30 mg/kg one hour before irradiation. The results are shown in FIG. 3.

FIG. 4 shows photographs of the results confirming collagen in the vascular endothelial sites of the lung of the mouse animal model by a trichrome staining method after treatment or non-treatment with CHIR-99021 or CHIR-98014 in the mouse animal model, which was followed by irradiation of the site of the lung.

As shown in FIG. 4, the blue sites where collagen is stained were hardly observed in the inner wall of the aorta of the control group without irradiation. However, it was observed that the site of blue color was relatively, remarkably increased in the inner wall of the aorta of the irradiation group (IR). Accordingly, it was confirmed that pulmonary fibrosis progressed after irradiating the site of the lung of the mouse. In contrast, in the mouse (IR+CHIR-98014) treated with CHIR-99021 (IR+CHIR-99021) or CHIR-98014, the blue sites of collagen *(i.e.,* a protein associated with pulmonary fibrosis) were remarkably reduced as compared to the irradiation group (IR).

FIG. 5 illustrates graphs which statistically show the level of expression of collagen *(i.e.,* a molecule associated with pulmonary fibrosis) by carrying out a trichrome staining method. Further, it was confirmed that there was a statistical significance in the effect between the experimental group, in which the phenomenon of fibrosis was increased due to irradiation, and the experimental group, in which the increase in the phenomenon of fibrosis was reduced according to the treatment with CHIR-99021 or CHIR-98014.

Based on the results above, it can be identified that CHIR-99021 or CHIR-98014 remarkably inhibited the phenomenon of fibrosis of pulmonary vascular endothelial cells caused by radiation.

### Example 4

### Inhibition test of GSK3 inhibitor on DNA damage caused by radiation

One of the side effects occurred after radiotherapy is DNA damage in normal cells around the irradiated site. When DNA gets damaged, the phosphorylation of γH2AX proceeds to recognize the damaged DNA and activate the repair signaling mechanism, and thus, DNA damage can be confirmed by staining with the γH2AX protein. Accordingly, γH2AX, which is known as a marker, was used for staining in order to measure the degree of DNA damage. The results are shown in FIG. 6.

FIG. 6 shows photographs of the results confirming DNA damage by a γH2AX immunostaining method after treatment or non-treatment with CHIR-99021 or CHIR-98014 at a concentration of 30 mg/kg in the mouse animal model one hour prior to irradiation, which was followed by irradiation of the site of the lung tissue of the mouse animal model with an intensity of 90 Gy.

As shown in FIG. 6, when compared with the normal tissue (No IR) through the γH2AX immunostaining, it was confirmed that the tissue of the 90Gy irradiation group (IR) showed considerable damage in DNA. In contrast, in the tissue of the mouse (IR+CHIR-98014) treated with CHIR-99021 (IR+CHIR-99021) or CHIR-98014, it was shown that the expression of γH2AX according to the DNA damage was remarkably reduced as compared to the non-treatment group (IR).

Based on such results of FIG. 6, it can be identified that the treatment with CHIR-99021 or CHIR-98014 remarkably reduced the DNA damage caused by radiation.

### Example 5

### Inhibition test of GSK3 inhibitor on tissue damage caused by Bleomycin

Bleomycin, which is commonly used as a therapeutic agent for various tumors including lymphoma, causes pulmonary damage and pulmonary fibrosis of tissue as one of the serious side effects, and thus, bleomycin is broadly used in preparing a model of acute pulmonary damage and pulmonary fibrosis in an experimental animal. Accordingly, a bleomycin-induced pulmonary fibrosis model was prepared to confirm the inhibitory effect of CHIR-99021 or CHIR-98014 on pulmonary damage and pulmonary fibrosis.

1.25 U/kg of bleomycin was mixed with 50 µL of a saline solution and then injected into the airway of a mouse. One hour before the bleomycin injection, 30 mg/kg of CHIR-99021 or CHIR-98014 was administered intraperitoneally. Three weeks after the bleomycin injection, the lung tissue of the mouse was fixed with 10% formalin, and a paraffin section was made, followed by identifying the lung tissue with hematoxylin and eosin staining and trichrome staining methods.

As shown in FIG. 7, it was confirmed that the tissue after three weeks of the bleomycin administration showed broad infiltration of inflammatory cells especially around the bronchus, deposition of collagen, fibrous nodules, and topical interstitial thickening of honeycomb appearance which is a representative characteristic of a bleomycin-induced pulmonary fibrosis model.

In contrast, it was confirmed that in the tissue of the mouse treated with CHIR-99021 or CHIR-98014, the pathological characteristics such as infiltration of inflammatory cells, deposition of collagen, fibrous nodules, interstitial thickening, *etc.* were remarkably inhibited as compared to the non-treatment group.

## Claims

1. A pharmaceutical composition for use in preventing or treating pulmonary fibrosis, comprising a GSK3 inhibitor of Formula 1 or Formula 2 below, or a pharmaceutically acceptable salt or a solvate thereof: 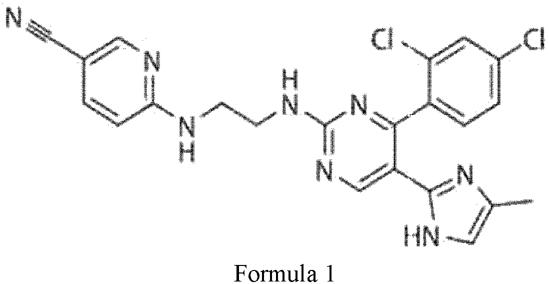 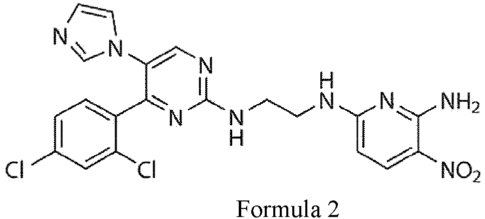 wherein the pulmonary fibrosis is induced by radiation exposure or drug therapy for anticancer treatment.

2. The pharmaceutical composition for use of claim 1 or 2, wherein the pharmaceutically acceptable salt is hydrochloride.

3. The pharmaceutical composition for use of claim 1, wherein the pharmaceutical composition is administered after or simultaneously with drug therapy for anticancer treatment.

4. The pharmaceutical composition for use of claim 1, wherein the pharmaceutical composition is administered after a radiation exposure.

5. The pharmaceutical composition for use of claim 1 or 2, wherein the pharmaceutical composition is administered before exposure to a radiation exposure or drug therapy for anticancer treatment.

## Patentansprüche

1. Pharmazeutische Zusammensetzung zur Verwendung bei der Vorbeugung oder Behandlung von Lungenfibrose, umfassend einen GSK3-Inhibitor der nachstehenden Formel 1 oder Formel 2, oder ein pharmazeutisch akzeptables Salz oder Solvat davon: 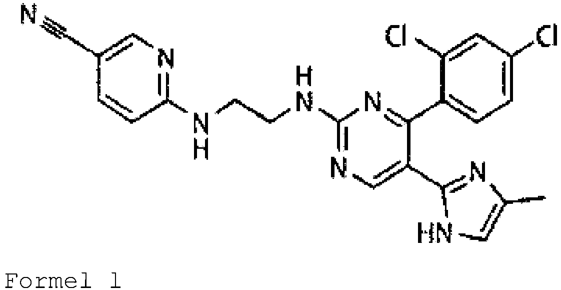 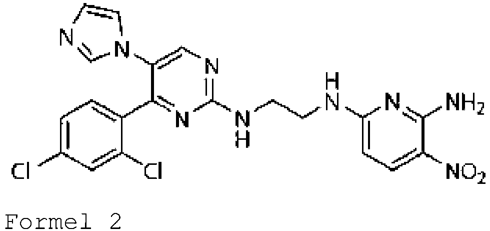 wobei die Lungenfibrose durch Strahlungsexposition oder Arzneimitteltherapie zur Antikrebsbehandlung induziert wurde.

2. Pharmazeutische Zusammensetzung zur Verwendung nach Anspruch 1, wobei das pharmazeutisch akzeptable Salz Hydrochlorid ist.

3. Pharmazeutische Zusammensetzung zur Verwendung nach Anspruch 1, wobei die pharmazeutische Zusammensetzung nach oder gleichzeitig mit der Arzneimitteltherapie zur Antikrebsbehandlung verabreicht wird.

4. Pharmazeutische Zusammensetzung zur Verwendung nach Anspruch 1, wobei die pharmazeutische Zusammensetzung nach einer Strahlungsexposition verabreicht wird.

5. Pharmazeutische Zusammensetzung zur Verwendung nach Anspruch 1 oder 2, wobei die pharmazeutische Zusammensetzung vor einer Strahlungsexposition oder Arzneimitteltherapie zur Antikrebsbehandlung verabreicht wird.

## Revendications

1. Composition pharmaceutique destinée à être utilisée dans la prévention ou le traitement de la fibrose pulmonaire, comprenant un inhibiteur de GSK3 de la formule 1 ou de la formule 2 ci-dessous, ou un sel pharmaceutiquement acceptable ou un solvate de celui-ci : 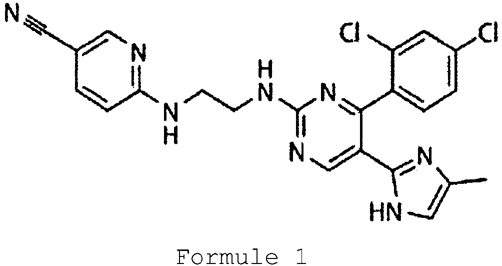 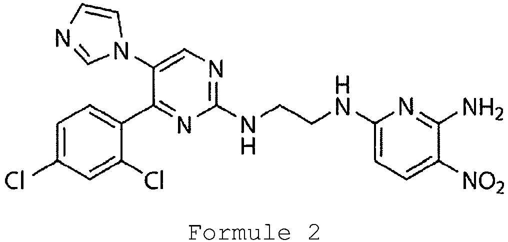 dans laquelle la fibrose pulmonaire est induite par une exposition à des rayonnements ou une thérapie médicamenteuse pour un traitement anticancéreux.

2. Composition pharmaceutique destinée à être utilisée selon la revendication 1, dans laquelle le sel pharmaceutiquement acceptable est le chlorhydrate.

3. Composition pharmaceutique destinée à être utilisée selon la revendication 1, dans laquelle la composition pharmaceutique est administrée après ou en même temps qu'une thérapie médicamenteuse pour un traitement anticancéreux.

4. Composition pharmaceutique destinée à être utilisée selon la revendication 1, dans laquelle la composition pharmaceutique est administrée après une exposition à des rayonnements.

5. Composition pharmaceutique destinée à être utilisée selon la revendication 1 ou 2, dans laquelle la composition pharmaceutique est administrée avant une exposition à une exposition à des rayonnements ou une thérapie médicamenteuse pour un traitement anticancéreux.
